# DEMANDE DE BREVET EUROPEEN

(11) **EP 1 600 152 A1**
(43) Date de publication de la demande: **30.11.2005**
(21) Numéro de dépôt: 05290720.1
(22) Date de dépôt: 01.04.2005
(51) Int. Cl.: A61K 7/48

(54) **Utilisation du lif en cosmétique et en dermatologie**

(30) Priorité: 26.05.2004 FR 0451034
(71) Demandeur: L'OREAL, 75008 Paris (FR)
(72) Inventeur: Fortunel, Nicolas, 91650 Breuillet (FR); Bernard, Dominique, 75015 Paris (FR); Ferraris, Corinne, 75017 Paris (FR); Regnier, Marcelle, 75018 Paris (FR)
(74) Mandataire: Allab, Myriam

(57) **Abrégé**

L'invention concerne l'utilisation dans une composition cosmétique ou pour la préparation d'une composition pharmaceutique d'une quantité efficace d'un composé choisi parmi le LIF (leukemia inhibitory factor ou facteur inhibiteur de leucémie), un analogue du LIF, un mimétique du LIF, un produit capable de stimuler l'expression du LIF endogène et leurs mélanges, comme agent destiné à maintenir et/ou stimuler le pouvoir régénératif d'une population de cellules souches cutanées et/ou de progéniteurs épidermiques indifférenciés humains.

L'invention porte également sur des compositions cosmétiques ou pharmaceutiques contenant un composé choisi parmi le LIF, un analogue du LIF, un mimétique du LIF, un produit capable de stimuler l'expression du LIF endogène et leurs mélanges, ainsi que sur des procédés cosmétiques de traitement des peaux sèches et/ou gercées et/ou âgées, ou sur des procédés cosmétiques de traitement de la chute des cheveux.

L'utilisation d'un composé choisi parmi le LIF, un analogue du LIF, un mimétique du L1F et un produit capable de stimuler l'expression du LIF endogène et leurs mélanges est également avantageuse dans le domaine de la régénération et/ou de la cicatrisation de la peau, en particulier pour le traitement des brûlures.

## Description

L'invention a pour domaine technique l'utilisation du LIF (leukemia inhibitory factor ou facteur inhibiteur de leucémie), d'un analogue du LIF ou d'un mimétique du LIF, en cosmétique et en dermatologie.

L'invention concerne notamment l'utilisation dans une composition cosmétique d'une quantité efficace d'un composé choisi parmi le LIF, un analogue du LIF, un mimétique du LIF, un produit capable de stimuler l'expression du LIF endogène et leurs mélanges, comme agent destiné à maintenir et/ou stimuler le pouvoir régénératif d'une population de cellules souches cutanées et/ou de progéniteurs épidermiques indifférenciés humains.

Par 'pouvoir régénératif d'une population de cellules souches cutanées et/ou de progéniteurs épidermiques indifférenciés humains' selon l'invention, on entend notamment (i) la capacité de ladite population de cellules à s'autorenouveler et/ou proliférer et/ou (ii) leur capacité à régénérer un épithélium pluristratifié, en particulier un épiderme pluristratifié et/ou tout ou partie des annexes cutanées (glandes sébacées, follicule pileux, ongles...).

Par 'population de cellules souches cutanées et/ou de progéniteurs épidermiques indifférenciés humains' selon l'invention, on entend une population de cellules souches somatiques adultes naturellement présentes dans la couche basale de l'épiderme, qui sont capables de s'autorenouveler et/ou proliférer et de générer un épithélium pluristratifié, en particulier un épiderme pluristratifié et/ou tout ou partie des annexes cutanées (glandes sébacées, follicule pileux, ongle...).

Par 'capacité d'une cellule à s'autorenouveler', on entend une cellule capable de se diviser pour donner deux cellules filles dont une au moins est identique à la cellule mère. A l'échelle d'une population cellulaire complexe, la notion d'autorenouvellement implique le maintien d'un compartiment de cellules de caractéristiques phénotypiques et fonctionnelles constantes au cours des divisions cellulaires successives. Il s'agira selon l'invention du maintien d'un compartiment composé de cellules souches cutanées et/ou de progéniteurs épidermiques indifférenciés, notamment caractérisés par un fort potentiel de prolifération et une capacité à générer un épithélium pluristratifié, en particulier un épiderme pluristratifié.

Par 'capacité d'une cellule à proliférer', on entend une cellule capable de se multiplier pour donner deux cellules filles, sans qu'il y ait nécessairement transmission des caractéristiques et du potentiel de la cellule mère à l'une au moins des deux cellules filles. La prolifération, qui peut ou non être associée au phénomène d'autorenouvellement, est susceptible de conduire à la diminution progressive ou la disparition du compartiment cellulaire d'intérêt au sein de la population cellulaire qui se multiplie. Selon l'invention, la prolifération cellulaire est susceptible de s'accompagner d'une diminution progressive et/ou de la disparition du compartiment des cellules souches cutanées et/ou des progéniteurs épidermiques indifférenciés, notamment caractérisés par un fort potentiel de prolifération et une capacité à générer un épithélium pluristratifié, en particulier un épiderme pluristratifié.

Cette population comprend notamment des cellules souches cutanées et/ou des progéniteurs épidermiques indifférenciés.
Le compartiment des cellules souches, situé le plus en amont de la hiérarchie du tissu cutané, est caractérisé par un fort potentiel d'autorenouvellement (capacité de multiplication à long-terme la plus importante), ainsi que par une multipotentialité (notamment capacité à produire un épithélium pluristratifié, en particulier des kératinocytes et/ou différents types cellulaires des annexes cutanées, telles que follicules pileux, glandes sébacées, ongles...).
Les progéniteurs épidermiques, issus desdites cellules souches, présentent un potentiel de multiplication qui peut également être important, mais généralement plus réduit que celui des cellules souches, et sont caractérisés par une capacité à générer un épithélium pluristratifié, en particulier un épiderme pluristratifié.

Une diminution quantitative et/ou qualitative du pool de cellules souches cutanées et/ou de progéniteurs épidermiques, ainsi qu'une altération de leur environnement cellulaire et moléculaire nécessaire à leur stimulation pourraient être une des causes possibles du vieillissement cutané.
D'où la recherche d'agents capables de maintenir et/ou augmenter ce pool de cellules souches cutanées et/ou de progéniteurs épidermiques, afin de favoriser le renouvellement épidermique et prévenir et/ou lutter contre les signes cutanés du vieillissement.

L'invention vise également l'utilisation d'un composé choisi parmi le LIF, un analogue du LIF, un mimétique du LIF, un produit capable de stimuler l'expression du LIF endogène et leurs mélanges pour la préparation d'une composition destinée à favoriser la régénération et/ou la cicatrisation de la peau, en particulier pour le traitement des brûlures.

L'invention porte également sur des compositions cosmétiques ou pharmaceutiques contenant au moins un composé choisi parmi le LIF, un analogue du LIF, un mimétique du LIF, un produit capable de stimuler l'expression du LIF endogène et leurs mélanges, et sur des procédés cosmétiques de traitement des peaux sèches et/ou gercées et/ou âgées ou sur des procédés cosmétiques de traitement de la chute des cheveux.

La peau constitue une barrière physique entre l'organisme et son environnement. Elle est constituée de deux tissus : l'épiderme et le derme.

Le derme fournit à l'épiderme un support solide. C'est également son élément nourricier. Il est principalement constitué de fibroblastes et d'une matrice extracellulaire elle-même notamment composée de collagène, d'élastine, de fibronectine, et d'une substance dite substance fondamentale, ces composants étant essentiellement synthétisés par les fibroblastes. On y trouve aussi des leucocytes, des mastocytes ou encore des macrophages tissulaires. Il contient également de vaisseaux sanguins et des fibres nerveuses.

L'épiderme est un épithélium pluristratifié desquamant, de 100 µm d'épaisseur en moyenne et est conventionnellement divisé en une couche basale contenant des cellules souches cutanées, des progéniteurs épidermiques indifférenciés, ainsi que des cellules engagées dans le processus de maturation/différenciation, une couche dite épineuse constituée de plusieurs couches de cellules polyédriques disposées sur les cellules basales, une couche dite granuleuse constituée de cellules aplaties contenant des inclusions cytoplasmiques, les grains de kératohyaline, et enfin une couche supérieure appelée couche cornée (ou *stratum corneum),* constituée de kératinocytes au stade terminal de leur différenciation, appelés cornéocytes. Ces derniers sont des cellules kératinisées momifiées, anucléées qui dérivent des kératinocytes. L'empilement des cornéocytes constitue la couche cornée qui assure entre autres la fonction de barrière de l'épiderme.

La différenciation épidermique suit un processus de maturation continu et orienté dans lequel les kératinocytes basaux se transforment en migrant pour aboutir à la formation de cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme. Celle-ci implique une régulation précise du nombre de cellules qui entrent dans le processus de différenciation et du nombre de cellules qui desquament. Au cours du processus normal de desquamation, seuls les cornéocytes les plus superficiels se détachent de la surface de l'épiderme.

Différents paramètres physiologiques sont associés à un « vieillissement » cutané. On sait notamment qu'au cours du vieillissement chronobiologique, l'épaisseur de l'épiderme se réduit. Le potentiel régénératif de l'épiderme devient moins important car les cellules de la couche basale comprenant notamment les cellules souches et les progéniteurs épidermiques, se divisent moins activement, conduisant notamment à un ralentissement et/ou une diminution du renouvellement épidermique. Il est également décrit qu'à la ménopause, le vieillissement cutané s'accélère et l'épaisseur de la peau diminue. Les femmes se plaignent de ce que leur peau tire et devient sèche, voire de l'apparition d'une xérose. Les déficits hormonaux associés à la ménopause s'accompagnent notamment d'une baisse d'activité métabolique, ce qui pourrait aboutir à une diminution de la prolifération des kératinocytes.

Un « vieillissement » cutané est également associé à des causes environnementales. Parmi les plus connues, on sait notamment que des expositions prolongées et/ou répétées au soleil aboutissent à des résultats assez similaires sur l'épiderme. Il s'agit du vieillissement photo-induit.

Les signes cutanés du vieillissement se traduisent notamment par un amincissement de la peau et/ou la perte de fermeté, d'élasticité et/ou de tonicité de la peau et/ou la formation de rides et ridules. Le microrelief de la peau, constitué de microdépressions à la surface de la peau, est plus accentué, et l'aspect de la peau moins lisse. Les rides et ridules apparaissent notamment au niveau du sillon nasogénien, de la patte d'oie, du front, autour de la bouche et au niveau du cou.

On sait aussi que dans le cas de certaines maladies comme le psoriasis, l'ichtyose, la peau présente des altérations associées à une prolifération et/ou une différenciation cellulaire altérée.

On comprend alors la nécessité de disposer d'agents capables de stimuler la multiplication des cellules épidermiques pour faciliter la régénération de l'épiderme et redonner à la peau un aspect jeune. On mesure également combien de tels actifs pourraient être utiles dans le cas de la cicatrisation et de la régénération de la peau, notamment pour le traitement des brûlures.

On connaît de l'art antérieur des composés capables de stimuler la prolifération des kératinocytes matures et favoriser ainsi le renouvellement épidermique, tels que par exemple les rétinoïdes, les dérivés de la vitamine A, les minéraux et oligoéléments.

Mais la Demanderesse vient de découvrir de façon inattendue que l'on pouvait également maintenir et/ou augmenter le pouvoir régénératif d'une population de cellules souches cutanées et/ou de progéniteurs épidermiques indifférenciés par l'utilisation du Leukemia Inhibitory Factor (LIF).

Par 'maintenir et/ou augmenter le pouvoir régénératif d'une population de cellules souches cutanées et/ou de progéniteurs épidermiques indifférenciés', on entend selon l'invention un agent capable notamment de:
(i) favoriser la capacité des cellules souches cutanées et/ou de progéniteurs épidermiques indifférenciés à s'autorenouveler et/ou à proliférer ;
(ii) et/ou de maintenir et/ou d'augmenter leur capacité à générer un épithélium pluristratifié, en particulier un épiderme pluristratifié et/ou à générer tout ou partie des annexes cutanées.

On entend également selon l'invention un agent capable de :
(i) favoriser la capacité des cellules souches cutanées et/ou de progéniteurs épidermiques indifférenciés à s'autorenouveler et/ou à proliférer
(ii) et/ou de maintenir et/ou d'augmenter leur capacité à générer un épithélium pluristratifié, en particulier un épiderme pluristratifié et/ou à générer tout ou partie des annexes cutanées,
en réponse à un stimulus exogène et/ou en réponse à un signal issu de l'environnement physiologique des cellules *in vivo.*

En effet, l'action du LIF, d'un analogue du LIF ou d'un mimétique du LIF pourra être directe, par stimulation du potentiel régénératif des cellules souches cutanées et/ou des progéniteurs épidermiques. Elle pourra également être indirecte, notamment par le biais de facteurs et/ou de signaux émis par les cellules réactives au LIF, et capables de stimuler d'une manière bénéfique les cellules voisines. Il s'agit par exemple de signaux transmis par contact direct entre les cellules, et/ou de régulations paracrines. La bonne santé de la peau ainsi que la régulation de son potentiel régénératif impliquent notamment ce type de 'dialogue' entre les fibroblastes du derme et les souches et/ou les progéniteurs épidermiques. Lesdites cellules seront dès lors plus réceptives à des stimuli exogènes et/ou à un signal issu de l'environnement physiologique des cellules *in vivo.*

L'homéostasie épidermique résulte notamment d'une balance finement régulée entre signaux mitogènes favorisant la division cellulaire et signaux anti-prolifératifs. Ces signaux résultent notamment de l'action des facteurs naturellement produits par les kératinocytes et/ou par les autres types cellulaires présents dans leur environnement, notamment sécrétés par les fibroblastes du derme.

Comme exemples de 'stimuli et/ou de signaux connus pour être impliqués dans l'homéostasie épidermique', on peut notamment citer :
- des facteurs de croissance, tels que les facteurs de croissance mitogéniques 'epidermal growth factor' (EGF) et 'keratinocyte growth factor' (KGF) (Cook *et al*., *J Cell Physiol.* 146: 277-289, 1991 ; Andreadis *et al*., *FASEB J*. 15: 898-906, 2001 ; Gamady *et al*., *J Cell Biochem.* 89: 440-449, 2003), ou encore le 'transforming growth factor-β1' (TGF-β1), facteur de croissance multifonctionnel notamment identifié pour son effet anti-prolifératif sur les kératinocytes *in vivo* et *in vitro* (Glick *et al*., *Proc Natl Acad Sci USA.* 90: 6076-6080, 1993 ; Van Ruissen *et al*., *J Cell Sci.* 107: 2219-2228, 1994; Cui *et al*., *Genes Dev.* 9: 945-955, 1995). Ces facteurs sont impliqués dans des boucles de régulation autocrines et paracrines fortement interactives et imbriquées, assurant à la fois le contrôle de la prolifération et celui de la différenciation (Reiss & Sartorelli, *Cancer Res.* 47: 6705-6709, 1987 ; Hertle *et al*., *J invest Dermatol.* 104: 260-265, 1995 ; Edmonson et *al*., *J Cell Physiol.* 179: 201-207, 1999 ; Yamasaki *et al*., *J Invest Dermatol.* 120: 1030-1037, 2003 ; Pasonen-Seppanen *et al*., *J Invest Dermatol.* 120: 1038-1044, 2003), et permettant ainsi une régulation précise du renouvellement et/ou de la maturation des kératinocytes ; et/ou
- des molécules pouvant compléter, moduler, et/ou interférer avec l'action des facteurs de croissance, telles que par exemple : la vitamine D et ses dérivés pour leur capacité à augmenter la sensibilité des kératinocytes à l'action mitogénique du KGF (Gamady *et al*., *J Cell Biochem.* 89: 440-449, 2003) ; l'acide rétinoïque pour sa capacité à moduler la prolifération et/ou la différenciation des kératinocytes (Choi & Fuchs, *Cell Regul.* 1: 791-809, 1990; Gibbs *et al*., *Arch Dermatol Res.* 288: 729-738, 1996; Chapellier *et al*., *EMBO J*. 21: 3402-3413, 2002), notamment par un mécanisme de sensibilisation des cellules à l'action de l'EGF et du TGF-β (Tong *et al*., *J Invest Dermatol.* 94: 126-131, 1990).

On comprend donc l'intérêt d'utiliser le LIF, un analogue du LIF, un mimétique du LIF, un produit capable de stimuler l'expression du LIF endogène ou leurs mélanges pour stimuler le pouvoir régénératif desdites cellules souches cutanées et/ou des progéniteurs épidermiques indifférenciés aptes à générer un épiderme pluristratifié et/ou tout ou partie des annexes cutanées, dans des compositions cosmétiques 'anti-âge' ou 'antichute', et/ou pour la préparation de compositions pharmaceutiques destinées à la régénération de la peau et/ou à la cicatrisation.

Cette nouvelle stratégie cible des populations de cellules situées plus en amont de la hiérarchie du tissu épidermique que toute population cellulaire composée de kératinocytes matures, différenciés, ou en voie de différenciation. Elle présente donc l'avantage d'ouvrir la possibilité de stimuler le renouvellement et/ou la régénération de tout ou partie des composants du tissu cutané, notamment de l'épiderme, d'une manière plus efficace et durable qu'en ciblant des populations cellulaires plus matures, différenciés, ou en voie de différenciation.

Le LIF est décrit dans le brevet US 6,261,548 comme un facteur capable de supprimer la prolifération de cellules myéloïdes leucémiques telles que les cellules murines M1, et à favoriser la différenciation des macrophages, suggérant son utilisation comme agent thérapeutique non prolifératif pour supprimer certaines formes de leucémies myéloïdes et pour modifier la fonction des macrophages impliqués dans la réponse aux infections.
Au niveau de la peau, le LIF est une cytokine naturellement produite par les kératinocytes *in vivo* et *in vitro* (Paglia *et al*., *Br J Dermatol.* 134: 817-823, 1996), et l'on sait que ce facteur est notamment impliqué dans le contrôle de processus inflammatoires associés à différentes pathologies cutanées comme le psoriasis (Bonifati *et al*., *Arch Dermatol Res.* 290: 9-13, 1998 ; Szepietowski *et al*., *J Dermatol.* 28: 115-122, 2001), ou certaines allergies (Szepietowski *et al*., *Contact Dermatitis.* 36: 21-25, 1997).

Mais à ce jour, il n'est ni décrit ni suggéré un effet du LIF, d'un analogue, d'un mimétique du LIF ou d'un produit capable de stimuler l'expression du LIF endogène sur le pouvoir régénératif des cellules souches cutanées en culture, ni leur utilisation dans des compositions cosmétiques 'anti-âge' ou 'antichute' ou des compositions pharmaceutiques topiques destinées à favoriser la cicatrisation et/ou la régénération de la peau, notamment pour des applications comme le traitement des brûlures.

L'invention a donc pour premier objet l'utilisation dans une composition cosmétique d'une quantité efficace d'un composé choisi parmi le LIF, un analogue du LIF, un mimétique du LIF, un produit capable de stimuler l'expression du LIF endogène et leurs mélanges comme agent destiné à maintenir et/ou stimuler le pouvoir régénératif d'une population de cellules souches cutanées et/ou de progéniteurs épidermiques indifférenciés humains.

En particulier, le LIF, un analogue du LIF, un mimétique du LIF, un produit capable de stimuler l'expression du LIF endogène ou leurs mélanges est notamment destiné à :
(i) favoriser la capacité des cellules souches cutanées et/ou de progéniteurs épidermiques indifférenciés à s'autorenouveler et/ou à proliférer ;
(ii) et/ou maintenir et/ou augmenter leur capacité à générer un épithélium pluristratifié, en particulier un épiderme pluristratifié et/ou à générer tout ou partie des annexes cutanées.

Par 'annexes cutanées', on entend notamment les follicules pileux, les glandes sébacées et les ongles.

Le LIF, un analogue du LIF, un mimétique du LIF, un produit capable de stimuler l'expression du LIF endogène ou leurs mélanges est également destiné à
(i) favoriser la capacité des cellules souches cutanées et/ou de progéniteurs épidermiques indifférenciés à s'autorenouveler et/ou à proliférer ;
(ii) et/ou maintenir et/ou augmenter leur capacité à générer un épithélium pluristratifié, en particulier un épiderme pluristratifié et/ou à générer tout ou partie des annexes cutanées ;
en réponse à un stimulus exogène et/ou en réponse à un signal issu de l'environnement physiologique des cellules *in vivo*.

Le LIF, un analogue du LIF, un mimétique du LIF, un produit capable de stimuler l'expression du LIF endogène ou leurs mélanges utilisé dans les compositions de l'invention est notamment destiné à :
- stimuler le renouvellement de l'épiderme et/ou de tout ou partie des annexes cutanées ; et favoriser notamment l'éclat du teint ;
- prévenir et/ou lutter contre les signes cutanés du vieillissement, en particulier prévenir un amincissement de la peau et/ou la perte de fermeté, d'élasticité et/ou de tonicité de la peau et/ou la formation de rides et ridules ; et/ou lisser le microrelief de la peau pour lui redonner un aspect jeune, lisse et ferme ;
- favoriser et/ou augmenter la fonction barrière de la peau et améliorer notamment l'hydratation de la peau; en particulier prévenir, améliorer et/ou remédier à des états de sécheresse de la peau et/ou des muqueuses, améliorer l'aspect esthétique et le confort de la peau (souplesse), limiter la formation de crevasses ou gerçures sur les lèvres, les mains, le visage ou le corps ;
- revitaliser le cuir chevelu et/ou régénérer la pousse des cheveux et/ou des poils.

L'invention concerne également l'utilisation d'une quantité efficace d'un composé choisi parmi le LIF, un analogue du LIF, un mimétique du LIF, un produit capable de stimuler l'expression du LIF endogène et leurs mélanges pour la préparation d'une composition destinée à la régénération et/ou la cicatrisation de la peau.

En particulier, la composition est destinée au traitement des brûlures, induites par une exposition excessive au soleil (érythème solaire) ou à la suite d'une exposition à une forte source de chaleur (feu, eau chaude...).

L'invention porte également sur l'utilisation d'une quantité efficace d'un composé choisi parmi le LIF, un analogue du LIF, un mimétique du LIF, un produit capable de stimuler l'expression du LIF endogène et leurs mélanges pour la préparation d'une composition destinée au traitement de pathologies associées à une desquamation altérée, telles que le psoriasis et l'ichtyose.

Le 'LIF' utilisé dans les compositions selon l'invention peut notamment être apporté sous la forme d'un LIF purifié, d'un LIF recombinant, d'un broyat ou extrait cellulaire contenant du LIF, d'un surnageant de culture de cellules contenant du LIF ou leurs mélanges.
De préférence on utilisera un LIF d'origine humaine.

On peut notamment utiliser:
- un LIF purifié à partir d'organes, de tissus, et/ou de cellules exprimant naturellement le LIF [ex : cellules de glande pituitaire (Ferrara *et al*., *Proc Natl Acad Sci USA.* 89: 698-702, 1992), fibroblastes du derme (Lorenzo *et al*., *Clin Immunol Immunopathol.* 70: 260-265, 1994), fibroblastes pulmonaires (Elias *et al*., *Am J Physiol.* 266: L426-435, 1994), placenta et endomètre (Kojima *et al*., *Biol Reprod.* 50: 882-887, 1994), cellules stromales de moelle osseuse (Lorgeot *et al*., *Cytokine.* 9: 754-758, 1997), cellules de rein (Morel *et al*., *Cytokine.* 12: 265-271, 2000), cardiomyocytes (Ancey *et al*., *Cytokine.* 18: 199-205, 2002)] ;
- un LIF recombinant, tel qu'obtenu en culture de microorganismes prokaryotes (ex : bactérie, *E. Coli*) ou de cellules eucaryotes (ex : levure, *Pichia pastoris, Saccharomyces),* sous forme ou non d'une protéine de fusion (ex : LIF recombinant humain commercialisé par Chemicon International Inc.) ;
- un broyat ou un extrait de cellules exprimant le LIF, en particulier un broyat ou un extrait de cellules nourricières (ex : fibroblastes murins de la lignée 3T3) exprimant le LIF, ou de cellules génétiquement modifiées pour exprimer le LIF, ou encore de cellules stimulées pour exprimer le LIF ;
- un surnageant de culture de cellules contenant du LIF, tel qu'un surnageant de culture de cellules 3T3 exprimant le LIF ;
ou leurs mélanges.

Comme 'produit capable de stimuler l'expression du LIF endogène', on peut citer par exemple l'IL-1β, cytokine décrite pour sa capacité à induire la synthèse et la sécrétion de LIF par les fibroblastes, cellules épithéliales et cellules musculaires lisses du poumon humain (Knight *et al*., *Am J Respir Cell Mol Biol.* 20: 934-841, 1999). On peut également citer le TNF-α, ainsi que l'analoque d'AMP cyclique 8-bromoadénosine 3' : 5' monophosphate (BBrcAMP), étudiés pour leur capacité à induire le promoteur du LIF dans une lignée murine de cellules issues du stroma médullaire (Gollner *et al*., *Cytokine.* 11: 656-663, 1999).
Selon une alternative, on peut utiliser tout principe actif capable au contraire de réprimer l'activité de voies de signalisation antagonistes de l'effet anti-différenciateur du LIF, et notamment celle impliquant la protéine Stat5. Il s'agit notamment d'oligonucléotides antisens, et/ou de méthodes d'interférence par des ARN de petite taille (siRNA), ou encore de modulateurs des activités kinases et phosphatases impliquées dans l'activation des molécules Stats.

Par 'analogue du LIF' selon l'invention, on entend notamment tout polypeptide LIF modifié ou tout fragment de polypeptide LIF ayant une activité LIF sur les cellules souches cutanées et/ou les progéniteurs épidermiques, c'est-à-dire qui est capable (i) de favoriser la capacité des cellules souches cutanées et/ou de progéniteurs épidermiques indifférenciés à s'autorenouveler et/ou à proliférer et (ii) de maintenir et/ou augmenter leur capacité à générer un épiderme pluristratifié.

Par 'polypeptide LIF modifié ayant une activité LIF', on entend notamment un polypeptide LIF ayant subi une ou plusieurs modifications, par exemple pour augmenter sa stabilité. Par 'modification', on entend toute substitution, délétion et/ou insertion d'un acide aminé ou d'un nombre réduit d'acides aminés, notamment par substitution d'acides aminés naturels par des acides aminés non naturels ou pseudoacides aminés à des positions telles que les modifications ne portent pas significativement atteinte à l'activité biologique du LIF.
Le polypeptide LIF modifié pourra être obtenu à partir d'une séquence peptidique de LIF humain choisie parmi la séquence Genbank AAA59217 (195 aa), la séquence GenBank AAA51699 (202 aa), et les séquences homologues.
Par 'séquence homologue', on entend une séquence identique à au moins 70%, de préférence au moins 85% et encore plus préférentiellement au moins 95% d'une séquence peptidique définie, chez la même espèce ou chez une espèce différente ; on parle alors de séquence peptidique orthologue.
On pourra également obtenir ce polypeptide LIF modifié à partir des séquences du gène ou de l'ADNc du LIF humain (GenBank M63420 J05436, J03261, X13967), des séquences du gène ou de l'ADNc du LIF murin (GenBank M63419 J05435, X06381, X12810, S73374), selon les techniques classiques de clonage et d'expression.

Par 'fragment de polypeptide LIF ayant une activité LIF', on entend notamment un fragment d'une séquence choisie parmi la séquence Genbank AAA59217 (195 aa), la séquence GenBank AAA51699 (202 aa) et les séquences homologues.
Ledit fragment aura notamment une taille suffisante pour reconstituer la structure tertiaire du LIF présentant les sites de liaison au LIF-R et à gp130.
Ce fragment de polypeptide pourra également être obtenu selon les techniques classiques de clonage et d'expression à partir des séquences du gène ou de l'ADNc du LIF humain (GenBank M63420, J05436, X13967, J03261), des séquences du gène ou de l'ADNc du LIF murin (GenBank X06381, M63419 J05435, X12810, S73374), et en particulier à partir des séquences codantes.

De tels analogues du LIF adaptés à la mise en oeuvre de l'invention pourront ainsi être sélectionnés selon le procédé comprenant les étapes suivantes :
a) on met en culture une préparation de kératinocytes ou de cellules souches cutanées et/ou de progéniteurs épidermiques indifférenciés (i) en présence ou (ii) en absence du produit à tester ;
b) on étudie au microscope les cellules cultivées selon (i) et (ii) ;
c) on mesure la capacité du produit à favoriser la capacité des cellules souches cutanées et/ou des progéniteurs épidermiques à s'autorenouveler et/ou proliférer en comparant le nombre de clones cellulaires à l'état indifférencié obtenus (i) en présence ou (ii) en absence du produit à tester ;
d) on sélectionne le produit pour lequel on obtient un nombre de clones cellulaires à l'état indifférencié augmenté en présence dudit produit par rapport au nombre de clones cellulaires à l'état indifférencié en absence dudit produit ;
e) on teste ensuite la capacité dudit produit à régénérer un épiderme pluristratifié selon les étapes suivantes :
   a. on ensemence sur un support de derme une préparation de kératinocytes (i) en présence ou (ii) en absence dudit produit à tester ;
   b. on observe au microscope la structure de l'épiderme reconstruit selon (i) et (ii) ;
   c. on sélectionne le produit pour lequel on obtient une structure de l'épiderme reconstruit améliorée en présence dudit produit par rapport à la structure de l'épiderme en absence dudit produit.

Par 'mimétique du LIF' selon l'invention, on entend notamment tout agoniste du récepteur du LIF (LIF-R) ou encore tout actif, extrait ou fraction d'extrait cellulaire capable d'activer la gp130 et/ou les voies de signalisation Jak/Stat et Ras/Map kinases, en particulier l'expression et/ou l'activité de Stat3.
On pourra par exemple utiliser des anticorps agonistes des récepteurs du LIF, des peptides de synthèse capables d'interagir avec les récepteurs du LIF et de les activer ou tout actif capable d'induire une activation des voies de signalisation impliquées dans la réponse au LIF, en particulier l'expression et/ou l'activité de Stat3, ou sur l'activation des Janus-associated tyrosine kinases (JAK).
En particulier, on pourra utiliser des agents capables d'induire la formation d'un hétérodimère LIFR-gp130, tels que par exemple la cytokine oncostatine M (OSM), le ciliary neurotrophic factor (CNTF) et la cardiotrophin-1 (CT-1), qui ont une forte homologie de structure tertiaire avec le LIF.
De tels mimétiques du LIF adaptés à la mise en oeuvre de l'invention pourront être sélectionnés par des tests classiques de liaison au LIF-R et d'activation de la gp130.

Ces analogues du LIF ou mimétiques du LIF pourront être d'origine naturelle ou synthétique.
Par 'origine naturelle', on entend un composé à l'état pur ou en solution à différentes concentrations, obtenu par différents procédés d'extraction à partir d'un tissu (peau...) d'origine naturelle, en particulier l'épiderme humain ou à partir d'extraits d'origine végétale.
Par 'origine synthétique', on entend un composé à l'état pur ou en solution à différentes concentrations, obtenu chimiquement ou par production dans un organisme après introduction dans cet organisme des éléments nécessaires à cette production.

On sait en effet que la réponse cellulaire au LIF implique un récepteur de faible affinité (glycoprotéine de 190 kD, gp 190) et un récepteur de forte affinité (glycoprotéine de 130 kD, gp 130) (Taupin *et al*., *J Biol Chem.* 276: 47975-47981, 2001), et en aval de ces récepteurs, les voies de signalisation Jak/Stat et Ras/Map kinases (Ernst *et al*., *J Biol Chem.* 274: 9729-9737, 1999 ; Burdon *et al*., *Trends Cell Biol.* 12: 432-438, 2002). La protéine de transduction du signal Stat3 joue un rôle prépondérant dans le maintien de l'état indifférencié des cellules ES murines en réponse au LIF (Niwa *et al*., *Genes Dev.* 12: 2048-2060, 1998; Matsuda *et al*., *EMBO J.* 18: 4261-4269, 1999), alors que l'expression de la protéine Stat5 est au contraire associée à un engagement de ces cellules vers la différenciation (Nemetz *et al*., *Differentiation.* 62: 213-220, 1998).

Selon une alternative, on pourra également utiliser dans une composition ou pour la préparation d'une composition selon l'invention, un récepteur du LIF (LIF-R) ou un produit capable de stimuler l'expression du LIF-R endogène, de préférence en association avec un LIF, un analogue du LIF, un mimétique du LIF ou un produit capable de stimuler l'expression du LIF.

La composition selon l'invention peut se présenter sous toutes les formes galéniques normalement utilisées dans les domaines cosmétique et dermatologique, adaptées à la voie orale ou topique, préférentiellement à la voie topique sur la peau et/ou les muqueuses ou le cuir chevelu.
La composition pourra être sous une forme adaptée au soin ou au maquillage de la peau et/ou des lèvres.

Une formulation adaptée à la voie orale peut être sous forme de dragées, gélules, gels, émulsions, comprimés, capsules ou solutions liquides notamment ampoules buvables, par exemple. En particulier, le(s) actif(s) selon l'invention peuvent être incorporés dans toutes autres formes de compléments alimentaires ou d'aliments enrichis, par exemple des barres alimentaires, ou des poudres compactées ou non.

De préférence la quantité efficace de LIF, d'un analogue du LIF, d'un mimétique du LIF, d'un produit capable de stimuler l'expression du LIF endogène ou leurs mélanges présente dans la composition est comprise entre 10⁻¹² % et 1 % du poids total de la composition, préférentiellement entre 10⁻⁹% et 0.1 % et encore plus préférentiellement entre 10⁻⁷ % et 0.01% du poids total de la composition.

Si l'on utilise un broyat ou un extrait cellulaire contenant du LIF, la quantité efficace dudit broyat ou dudit extrait présente dans la composition est comprise entre 10⁻⁹ % et 1 % du poids total de la composition, préférentiellement entre 10⁻⁶ % et 0.1 % et encore plus préférentiellement entre 10⁻⁴ % et 0.01% du poids total de la composition.

L'invention porte également sur une composition adaptée à une application topique sur la peau comprenant, dans un milieu physiologiquement acceptable, au moins une quantité efficace d'un composé choisi parmi le LIF, un analogue du LIF, un mimétique du LIF, un produit capable de stimuler l'expression du LIF endogène ou leurs mélanges.

Par 'milieu physiologiquement acceptable' selon l'invention, on entend un milieu compatible avec la peau et/ou ses phanères (cils, ongles, cheveux) et/ou les muqueuses (lèvres).

La composition selon l'invention est une composition cosmétique ou une composition pharmaceutique, de préférence une composition cosmétique.
La composition pharmaceutique sera de préférence une composition dermatologique.

La quantité efficace de LIF, d'un analogue d LIF, d'un mimétique du LIF, d'un produit capable de stimuler l'expression du LIF endogène ou leurs mélanges présente dans la composition est préférentiellement comprise entre 10⁻¹² % et 1 % du poids total de la composition, préférentiellement entre 10⁻⁹ % et 0.1% et encore plus préférentiellement entre 10⁻⁷ % et 0.01% du poids total de la composition.
Si l'on utilise un broyat ou un extrait cellulaire contenant du LIF, la quantité efficace dudit broyat ou dudit extrait présente dans la composition est comprise entre 10⁻⁹ % et 1 % du poids total de la composition, préférentiellement entre 10⁻⁶ % et 0.1 % et encore plus préférentiellement entre 10⁻⁴ % et 0.01% du poids total de la composition.

La composition peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

On pourra également formuler le LIF, un analogue du LIF, un mimétique du LIF, un produit capable de stimuler l'expression du LIF endogène ou leurs mélanges dans des compositions adaptées au ciblage dans les couches profondes de l'épiderme, en particulier au ciblage dans les couches basales de l'épiderme ou au niveau de l'unité pilo-sébacée. Par exemple, le LIF pourra être (i) encapsulé dans un enrobage tel que des microsphères, des nanosphères, des oléosomes ou des nanocapsules, ou (ii) compartimenté dans une phase grasse contenant les principaux constituants du sébum (squalène, triglycérides, cires aliphatiques, cires de cholestérol et cholestérol libre), ou des constituants de structure présents dans des proportions similaires à celles présentes dans le sébum.
Les particules appelées nanoparticules sont en effet capables de traverser les couches superficielles du *stratum corneum* et/ou de l'ostium folliculaire et de pénétrer dans les couches de l'épiderme.
L'intérêt d'une composition dont la phase grasse mime la composition du sébum, est de permettre une meilleure disponibilité de l'actif au niveau de l'organe cible, c'est-à-dire de la glande sébacée.
La composition peut avoir par exemple l'aspect d'une crème blanche ou colorée, d'une pommade, d'un lait, d'une lotion, d'un gel, d'un sérum, d'une pâte, d'une mousse, ou sous forme solide (ex : stick) pour une application sur la peau et/ou les muqueuses, telles que les lèvres.
Elle peut également se présenter sous la forme d'une lotion de type solution aqueuse, hydroalcoolique ou huileuse, d'une émulsion huile-dans-eau ou eau-dans-huile ou multiple, d'un gel aqueux ou huileux ou toute autre forme adaptée à une application sur la peau, les muqueuses ou le cuir chevelu.

De façon connue, la composition utilisée selon l'invention peut contenir également les adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées du LIF.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale ou synthétique (huile de vaseline, isohexadécane), les huiles d'origine végétale (huile d'amande d'abricot, fraction liquide de beurre de karité, d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme conservateurs, on peut citer les esters d'acide para-hydroxybenzoïque, l'octane 1,2-diol, l'iodo-3 propynyl-2 butyl carbamate, le phénoxyéthanol et le gluconate de chlorhexidine.

Comme charges, on peut citer par exemple, les particules de polyamide (Nylon) ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Comme 'solvants', on peut citer les solvants organiques hydrophiles, les solvants organiques lipophiles, les solvants amphiphiles ou leurs mélanges.
Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, des polyéthylèneglycols éventuellement oxyéthylénés, des polyols tels que le propylèneglycol, l'isoprèneglycol, le butylène glycol, le glycérol, le sorbitol et ses dérivés, les éthers de glycol et les éthers de propylène glycol. Comme solvants organiques amphiphiles, on peut citer des polyols tels que des dérivés de propylèneglycol.
Comme solvants organiques lipophiles, on peut citer par exemple les esters gras.

Comme 'actif hydrophile ou lipophile', on peut citer les agents hydratants, les agents apaisants, les agents dépigmentants, les agents anti-glycation, les inhibiteurs de NO-synthase, les agents stimulant la synthèse de macromolécules dermiques ou épidermiques et/ou empêchant leur dégradation, les agents stimulant la prolifération des fibroblastes et/ou des kératinocytes ou stimulant la différenciation des kératinocytes, les agents dermo-décontractants, les agents tenseurs, les agents anti-pollution et/ou anti-radicalaires, les agents photoprotecteurs, les agents réparateurs et/ou cicatrisants, les agents antipelliculaires et leurs mélanges.

Des exemples de tels composés additionnels sont donnés ci-dessous.

Par "agent hydratant", on entend :
- soit un composé agissant sur la fonction barrière, en vue de maintenir l'hydratation du *stratum corneum,* ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; les C-glycosides ;
- soit un composé augmentant directement la teneur en eau du *stratum corneum,* tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine, l'urée et ses dérivés ;
- soit un composé activant les glandes sébacées tel que la DHEA, ses dérivés 7-oxydés et/ou 17-alkylés, les sapogénines et la vitamine D et ses dérivés ;
- soit un composé agissant sur les activités hydrolytiques liées notamment à la desquamation, tels que l'HEPES et les dérivés sulfoniques, l'acide jasmonique et ses dérivés.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Par "agent anti-glycation", on entend un composé prévenant et/ou diminuant la glycation des protéines de la peau, en particulier des protéines du derme telles que le collagène.
Des exemples d'agents anti-glycation sont les extraits végétaux de la famille des Ericaceae, tels qu'un extrait de myrtille *(Vaccinium angustifolium) ;* l'ergothionéine et ses dérivés ; et les hydroxystilbènes et leurs dérivés, tels que le resvératrol et le 3,3', 5,5'-tétrahydroxystilbène.

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial *Ginkgo biloba* extrait standard.

Parmi les actifs stimulant les macromolécules du derme ou empêchant leur dégradation, on peut citer ceux qui agissent :
- soit sur la synthèse du collagène, tels que les extraits de *Centella asiatica* ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de *Saccharomyces Cerevisiae* commercialisé par la société LSN sous la dénomination commerciale Cytovitin® ; et l'extrait d'algue *Macrocystis pyrifera* commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune *Padina pavonica* commercialisé par la société ALBAN MÜLLER sous la dénomination commerciale HSP3® ; et l'extrait de *Saccharomyces cerevisiae* disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton *Salina* commercialisé par la société SEPORGA sous la dénomination commerciale GP4G® ;
   l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9 . On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse *(Pisum sativum*) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillaggrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® ; et l'extrait de zooplancton *Salina* commercialisé par la société SEPORGA sous la dénomination commerciale GP4G®.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de *Solanum tuberosum* commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl® ; et les lignanes tels que le sécoisolaricirésinol.

La composition selon l'invention peut comprendre des agents dermo-décontractants, parmi lesquels on peut citer en particulier l'alvérine et ses sels, notamment le citrate d'alvérine, les sapogénines telles que la diosgénine et les extraits naturels en contenant (tels que les extraits de *Wild Yam),* certaines amines secondaires et tertiaires carbonylées, des sels organiques ou inorganiques de métaux, en particulier le gluconate de manganèse, l'adénosine, ainsi que l'hexapeptide argireline R commercialisé par la société LIPOTEC. On peut également citer l'extrait de *Boswellia serrata* et certaines compositions parfumantes à effet dermo-décontractant.

Par "agent tenseur", on entend un composé capable d'exercer une traction sur la peau, qui a pour effet d'estomper temporairement les irrégularités de la surface de la peau, telles que les rides et ridules.
Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(3) les silicates mixtes, notamment les phyllosilicates et en particulier les Laponites,
(4) les microparticules de cire, choisies par exemple parmi les cires de *Carnauba,* de *Candelila* ou *d'Alfa,*
(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou *eichornia crassipes* ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (*Eichornia crassipes*) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Comme indiqué précédemment, la composition selon l'invention peut également renfermer des agents photoprotecteurs ou filtres UVA et/ou UVB, sous forme de composés organiques ou inorganiques, ces derniers étant éventuellement enrobés pour les rendre hydrophobes.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Les agents réparateurs et/ou cicatrisants peuvent être choisis parmi les vitamines A et dérivés, vitamine B3, pro-vitamine B5, vitamine C et dérivés, vitamine E ; les oligoéléments zinc, cuivre, manganèse, magnésium ; les extraits végétaux de *centella asiatica, mimosa tenuiflora,* allantoine, *aloe vera ;* l'acide hyaluronique et les alginates, et leurs mélanges.

Les agents antipelliculaires peuvent être choisis parmi les agents antifongiques et/ou antibactériens. On peut citer : le sel de zinc de pyridinethione ou omadine de zinc, les dérivés de 1-hydroxy-2-pyrrolidone, les trihalogéno carbamide, le triclosan, les composés azolés tels que le climbazole, le kétoconazole, le clotrinazole, l'econazole, l'isoconazole et le miconazole, les polymères antifongiques tels que l'amphotéricine B ou la nystatine, les sulfures de sélénium.

L'invention porte également sur un procédé cosmétique de traitement des peaux sèches et/ou gercées et/ou âgées, caractérisé en ce que l'on administre ou que l'on applique sur la peau ou les muqueuses une composition cosmétique telle que définie précédemment.

L'application sera de préférence localisée au niveau des zones sèches et/ou gercées de la peau du visage, du corps ou des lèvres, et au niveau des zones de rides du visage (front, patte d'oie, sillons nasogéniens) et du cou.

Un autre objet est un procédé cosmétique de traitement de la chute des cheveux, caractérisé en ce que l'on administre ou que l'on applique sur le cuir chevelu une composition cosmétique telle que définie précédemment.
Ces compositions sont destinées à être utilisées dans les cas de chute de cheveux, que cette chute soit d'origine naturelle ou médicamenteuse, c'est-à-dire suite à l'absorption de médicaments ayant pour effet secondaire une chute de cheveux.

L'invention sera maintenant illustrée par les exemples non limitatifs suivants.

### FIGURES

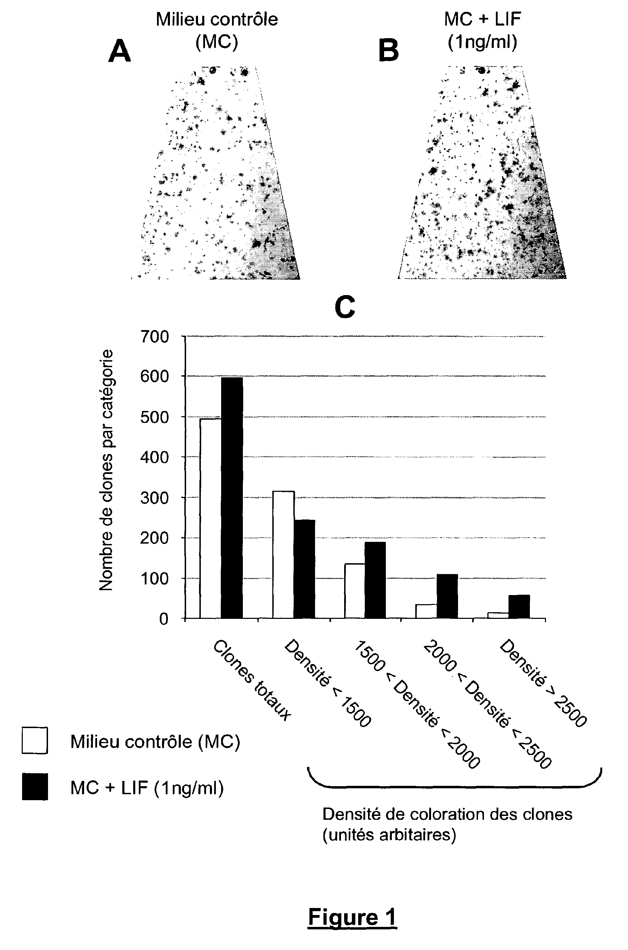

La **Figure 1** présente une analyse de l'effet du LIF sur la croissance clonogénique des cellules souches et/ou des progéniteurs épidermiques. Clones cellulaires obtenus à partir de cellules au passage 1 cultivées pendant 8 jours en absence de LIF exogène, puis fixés et colorés (**A**) ; Clones cellulaires obtenus à partir de kératinocytes au passage 1 cultivés pendant 8 jours en présence de LIF exogène à 1ng/ml, puis fixés et colorés (**B**) ; analyse semi-quantitative de la densité des clones obtenus en condition contrôle et en présence de LIF exogène (**C**).

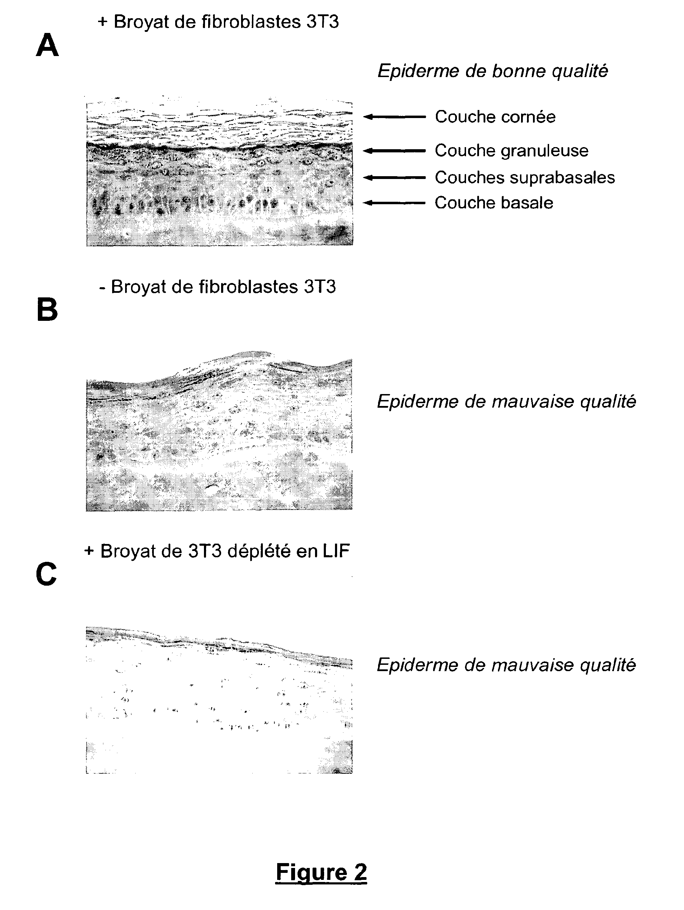

La **Figure 2** présente des analyses histologiques d'un épiderme reconstruit obtenu en présence ou non d'un broyat de fibroblastes 3T3 déplété ou non en LIF. Coupe histologique d'un épiderme reconstruit obtenu en présence de broyat de 3T3 non déplété en LIF (contrôle positif) (**A**) ; Coupe histologique d'un épiderme reconstruit obtenu en absence de broyat de 3T3 (contrôle négatif) (**B**) ; Coupe histologique d'un épiderme reconstruit obtenu en présence de broyat de 3T3 déplété en LIF (condition expérimentale) (**C**).

### EXEMPLES

### Exemple 1 : Croissance des cellules souches et/ou progéniteurs épidermiques indifférenciés optimisée en présence de LIF.

Des kératinocytes ont été isolés à partir d'un prélèvement cutané adulte (plastie mammaire).

Après élimination du tissu sous-cutané à l'aide d'un scalpel, le prélèvement cutané est découpé en fragments d'environ 5mm × 5mm, puis décontaminé par un traitement antibiotique [Gentamycine (Life Technologies), 3 bains successifs de 10 minutes dans du milieu de culture DMEM (Life Technologies)]. Pour permettre la séparation du derme de l'épiderme, le prélèvement est ensuite soumis à un traitement protéolytique [dispase (Boehringer, Roche Diagnostics) + trypsine (Gibco, Invitrogen) 1 nuit à 4°C]. L'épiderme est ensuite séparé du derme par dissection. Les fragments d'épiderme séparés du tissu dermique sont placés dans une solution de trypsine 0,05%-EDTA 0,02% (Gibco, Invitrogen) (15 minutes à 37°C). La préparation est agitée périodiquement pour favoriser la dissociation des cellules. L'effet de la trypsine est ensuite neutralisé par l'ajout d'un milieu de culture contenant 10% sérum (DMEM+10%sérum). Après neutralisation de la trypsine (DMEM+10%sérum), la préparation cellulaire est homogénéisée mécaniquement (pipettages), puis filtrée. La suspension cellulaire est lavées, puis remises en suspension dans du milieu KGM (Clonetics). Les cellules en suspension sont comptées au microscope à l'aide d'une cellule de Malassez. La viabilité des échantillons est estimée par la méthode d'exclusion au bleu Trypan (Life Technologies).

Une étape d'enrichissement en cellules souches et/ou progéniteurs épidermiques indifférenciés a été réalisée par une étape d'adhésion rapide sur un substrat de collagène de type **I** (sélection d'une population à forte capacité d'adhésion, Adh⁺⁺⁺).

Les cellules souches et/ou progéniteurs épidermiques indifférenciés peuvent en effet être séparées des kératinocytes plus matures sur la base de leur propriété d'adhésion rapide. Cette étape permet un pré-enrichissement de la préparation en cellules souches et/ou progéniteurs épidermiques indifférenciés.

La méthode d'enrichissement par adhésion sur collagène est décrite dans la demande WO 03/038073 et la publication scientifique (Fortunel *et al*., *J Cell Sci,* 116 : 4043-4052, 2003).

La suspension cellulaire est placée dans des flacons de culture « recouverts » avec du collagène de type I [l'adsorption du collagène sur le support d'adhésion est réalisée par dépôt d'une solution liquide de collagène I (Sigma Chemical) diluée d'un facteur 2 dans du PBS, pendant au moins 45 minutes, puis séchage après élimination du surplus], à une densité de 150 000 à 200 000 cellules/cm². Après 12 à 15 minutes, les kératinocytes n'ayant pas adhéré sont éliminés par lavage en tampon PBS. Les cellules adhérentes, dites Adh⁺⁺⁺, ainsi sélectionnées sont détachées du support par une trypsination douce (trypsine 0,05%-EDTA 0,02% (Gibco, Invitrogen) pendant 3 à 5 minutes à 37°C). Après neutralisation de la trypsine (DMEM+10%sérum), les cellules sont récupérées, lavées, puis remises en suspension dans du milieu de culture (dans le cas présent, milieu KGM). La fraction composée des cellules adhérentes, sélectionnée par cette méthode, représente environ 5 à 10% des kératinocytes totaux de l'épiderme.

Les cultures, initiées à partir de cellules Adh⁺⁺⁺, ont été réalisées en milieu de culture semi-défini (KGM Bullet Kit, Clonetics, Cambrex Bio Science Inc.), en absence de fibroblastes nourriciers, et à faible densité (ensemencements à 2400 cellules/cm²) afin d'obtenir des clones cellulaires isolés quantifiables. Au premier repiquage (passage 1), les cultures ont été divisées en 2 lots : 1) condition identique à celle décrite ci-dessus ; 2) addition de LIF à une concentration de 1ng/ml (LIF recombinant humain commercialisé par Chemicon International Inc.). Après 8 jours de culture, les cultures ont été fixées (éthanol 70%) et colorées (éosine et Giemsa) afin d'analyser les caractéristiques des clones cellulaires obtenus dans ces 2 conditions de culture (**Figure 1**).

L'analyse comparative de cultures effectuées en absence ou en présence de LIF montre que ce facteur permet d'optimiser la croissance des cellules souches et/ou progéniteurs épidermiques indifférenciés issus d'une culture de cellules Adh⁺⁺⁺.

L'observation macroscopique indique une augmentation du nombre de clones cellulaires de coloration dense de grande taille (**Figure 1 A,B**). L'observation microscopique indique que ces clones dont le développement est favorisé sont essentiellement constitués de cellules de petite taille présentant des caractéristiques morphologiques associées à l'état indifférencié des cellules souches et/ou des progéniteurs épidermiques, critère traduisant une « jeunesse » plus importante des cultures traitées par le LIF. L'augmentation du nombre de clones denses de grande taille en réponse au LIF est confirmée par analyse d'image informatisée, comme représenté à l'histogramme présenté **Figure 1C**.

L'utilisation du LIF dans un système de culture permet donc de favoriser la multiplication d'une population de cellules souches épidermiques et/ou de progéniteurs épidermiques humains indifférenciés.

### Exemple 2 : Effet positif du LIF sur la reconstitution épidermique.

Nous avons observé qu'un broyat de fibroblastes 3T3 favorise la reconstitution épidermique à partir de cellules souches et/ou de progéniteurs épidermiques cultivées *in vitro* et conservés sous forme congelée. L'épiderme reconstruit est de bonne qualité : il présente une organisation cellulaire et une stratification proche de celles d'un épiderme natif (**Figure 2A**).

Afin de tester l'hypothèse d'une implication du LIF dans cette propriété, une déplétion du LIF par immuno-précipitation a été réalisée, puis l'activité du broyat exempt de LIF a été comparée à celle du broyat non déplété.

La déplétion du broyat de 3T3 en LIF est réalisée à l'aide d'un anticorps polyclonal produit chez le lapin (Ac anti-LIF, Santa Cruz, réf. SC-20087). Le broyat de 3T3 est incubé avec l'Ac anti-LIF en excès à 4°C pendant plusieurs heures afin d'assurer une bonne fixation du LIF sur l'Ac. L'Ac libre et lié au LIF est ensuite 'trappé' par ajout de billes de sépharose sur lesquelles sont adsorbées des protéines G (les protéines G ont une forte affinité pour les domaines Fc des Ac). Une centrifugation permet séparer les billes sur lesquelles le LIF est retenu du broyat déplété en LIF.

La capacité des cellules souches épidermiques issues d'une banque congelée (isolées à partir d'un prélèvement cutané mammaire) à générer un épiderme reconstruit a été évaluée en présence de broyat de fibroblastes 3T3 non déplété en LIF (témoin positif), en absence de broyat de 3T3 (témoin négatif), et en présence de broyat de 3T3 déplété en LIF (condition expérimentale). Les caractéristiques histologiques des épidermes reconstruits obtenus dans chaque condition ont été comparées sur des coupes fixées et colorées (**Figure 2**).

Alors que les cultures organotypiques supplémentées en broyat de fibroblastes 3T3 (non déplété en LIF) permettent l'obtention d'épidermes reconstruits de bonne qualité (**Figure 2A**), c'est-à-dire une organisation cellulaire et une stratification proches de celles d'un épiderme natif, les cultures réalisées en absence de broyat ne permettent d'obtenir que des épidermes ne possédant pas les caractéristiques histologiques requises (**Figure 2B**).

Les épidermes obtenus en absence de broyat de 3T3 présentent notamment les anomalies suivantes :
- Vacuoles et espaces intercellulaires importants ;
- Mauvaise stratification ;
- Couche basale pauvre en cellules ;
- Cellules suprabasales très étirées ;
- Mauvaise orientation des cellules basales (parallèles à la matrice) ;
- Peu ou pas de couche granuleuse ;
- Couche cornée de faible épaisseur.

Les cellules de la condition expérimentale, cultivées en présence de broyat de 3T3 déplété en LIF, se comportent d'une manière similaire à celles cultivées en absence de broyat (**Figure 2C**), ce qui indique que la déplétion du LIF entraîne une perte des propriétés bénéfiques du broyat sur la reconstruction épidermique.

Ainsi , l'utilisation de LIF, outre sa capacité à favoriser la multiplication desdites cellules dans un état indifférencié comme montré à l'exemple 1, permet de maintenir le potentiel organogénique desdites cellules, c'est à dire leur capacité à générer un épiderme reconstruit de qualité, présentant des caractéristiques proches de celles de l'épiderme natif.

### Exemple 3 : Compositions.

| Composition cosmétique pour peaux sèches | |
|---|---|
| **LIF recombinant de l'ex.1** | **10**^{**-6**} **%** |
| Acide n-octanoyl-5-salicylique | 1 % |
| Méthylparaben | 0,1 % |
| Propylparaben | 0,1 % |
| Lanoline | 5 % |
| Huile de vaseline | 4 % |
| Huile de sésame | 4 % |
| Alcool cétylique | 5 % |
| Monostéarate de glycérol | 2 % |
| Triéthanolamine | 1 % |
| Propylène glycol | 5 % |
| Carbomer 940 | 0,1 % |
| Eau | qsp 100 % |

| Crème cosmétique anti-rides | |
|---|---|
| **Surnageant de culture de cellules 3T3 exprimant le LIF** | **10**^{**-3**} **%** |
| Stéarate de glycérol (émulsionnant) | 2,00 % |
| Polysorbate 60 (Tween 60 vendu par la société ICI) (émulsionnant) | 1,00 % |
| Acide stéarique | 1,40 % |
| Triéthanolamine (neutralisant) | 0,70 % |
| Carbomer (Carbopol 940 vendu par la société Goodrich) | 0,40 % |
| Fraction liquide de beurre de karité | 12,00 % |
| Perhydrosqualène | 12,00 % |
| Conservateur | 0,30 % |
| Parfum | 0,50 % |
| Antioxydant | 0,05 % |
| Eau | qsp 100 % |

| Stick de soin pour lèvres gercées | |
|---|---|
| **Surnageant de culture de cellules 3T3 exprimant le LIF** | **10**^{**-3**} **%** |
| Lanolide | 13 % |
| Cire microcristalline | 20 % |
| Ozokerite | 5 % |
| Huile polaire (huile de ricin) | 32 % |
| Huile non polaire (polydécène) | 27 % |
| Palmitate d'ascorbyle | 1 % |
| Vitamine E | 1 % |
| Parfum | 1 % |

| Lotion non rincée antichute | |
|---|---|
| **LIF recombinant de l'ex.1** | **1 µg** |
| Acide linoléique | 0,1 g |
| Propylène glycol | 22,8 g |
| Ethanol 95° | 55,1 g |
| Eau purifiée | qsp 100 g |

| Crème de soin de l'érythème | |
|---|---|
| **LIF recombinant de l'ex.1** | **10**^{**-6**}**%** |
| Stéarate de glycérol | 2,00% |
| Polysorbate 60 | 1,00% |
| Acide stéarique | 1,40% |
| Acide glycyrrhétinique | 2,00% |
| Triéthanolamine | 0,70% |
| Carbomer | 0,40% |
| Extrait d'aloé vera | 2,00% |
| Huile de tournesol | 10,00% |
| Antioxydant | 0,05% |
| Parfum | 0,50% |
| Conservateur | 0,30% |
| Eau | qsp 100% |

## Revendications

1. Utilisation dans une composition cosmétique d'une quantité efficace d'un composé choisi parmi le LIF, un analogue du LIF, un mimétique du LIF, un produit capable de stimuler l'expression du LIF endogène et leurs mélanges, comme agent destiné à maintenir et/ou stimuler le pouvoir régénératif d'une population de cellules souches cutanées et/ou de progéniteurs épidermiques humains indifférenciés.

2. Utilisation selon la revendication 1, **caractérisée en ce que** le LIF, un analogue du LIF, un mimétique du LIF, un produit capable de stimuler l'expression du LIF endogène et leurs mélanges est destiné à :
(i) favoriser la capacité des cellules souches cutanées et/ou de progéniteurs épidermiques indifférenciés à s'autorenouveler et/ou à proliférer ;
(ii) et/ou maintenir et/ou augmenter leur capacité à générer un épithélium pluristratifié, en particulier un épiderme pluristratifié et/ou tout ou partie des annexes cutanées.

3. Utilisation selon l'une quelconque des revendications 1 ou 2 , **caractérisée en ce que** le LIF, un analogue du LIF, un mimétique du LIF, un produit capable de stimuler l'expression du LIF endogène et leurs mélanges, est destiné à stimuler le renouvellement de l'épiderme et/ou de tout ou partie des annexes cutanées.

4. Utilisation selon l'une quelconque des revendications 1 à 3, **caractérisée en ce que** le LIF, un analogue du LIF, un mimétique du LIF, un produit capable de stimuler l'expression du LIF endogène et leurs mélanges, est destiné à prévenir et/ou lutter contre les signes cutanés du vieillissement, en particulier prévenir et/ou lutter contre l'amincissement de la peau et/ou la perte de fermeté, d'élasticité et/ou de tonicité de la peau et/ou la formation de rides et ridules.

5. Utilisation selon l'une quelconque des revendications 1 à 3, **caractérisée en ce que** le LIF, un analogue du LIF, un mimétique du LIF, un produit capable de stimuler l'expression du LIF endogène et leurs mélanges, est destiné à favoriser la fonction barrière de la peau, en particulier à favoriser l'hydratation de la peau.

6. Utilisation selon l'une quelconque des revendications 1 à 3, **caractérisée en ce que** le LIF, un analogue du LIF, un mimétique du LIF, un produit capable de stimuler l'expression du LIF endogène et leurs mélanges, est destiné à prévenir et/ou limiter la formation de gerçures sur les lèvres, les mains, le visage et/ou le corps.

7. Utilisation selon l'une quelconque des revendications 1 à 3, **caractérisée en ce que** le LIF, un analogue du LIF, un mimétique du LIF, un produit capable de stimuler l'expression du LIF endogène et leurs mélanges, est destiné à revitaliser le cuir chevelu et/ou régénérer la pousse des cheveux et/ou des poils.

8. Utilisation d'une quantité efficace d'un composé choisi parmi le LIF, un analogue du LIF, un mimétique du LIF, un produit capable de stimuler l'expression du LIF endogène et leurs mélanges, pour la préparation d'une composition destinée à favoriser la cicatrisation de la peau et/ou la régénération de la peau, en particulier dans le cas du traitement des brûlures.

9. Utilisation d'une quantité efficace d'un composé choisi parmi le LIF, un analogue du LIF, un mimétique du LIF, un produit capable de stimuler l'expression du LIF endogène et leurs mélanges, pour la préparation d'une composition destinée au traitement de pathologies associées à une desquamation altérée de la peau.

10. Utilisation selon l'une quelconque des revendications 1 à 9, **caractérisée en ce que** le LIF est un LIF d'origine humaine apporté dans la composition sous la forme d'un LIF purifié, d'un LIF recombinant, d'un broyat ou extrait cellulaire contenant du LIF, d'un surnageant de culture de cellules contenant du LIF, ou leurs mélanges.

11. Utilisation selon l'une quelconque des revendications 1 à 9, **caractérisée en ce que** l'analogue du LIF est choisi parmi un polypeptide LIF modifié ou un fragment de polypeptide LIF ayant une activité LIF sur les cellules souches cutanées et/ou les progéniteurs épidermiques, capable (i) de favoriser la capacité des cellules souches cutanées et/ou de progéniteurs épidermiques indifférenciés à s'autorenouveler et/ou à proliférer ; et/ou maintenir et/ou augmenter leur capacité à générer un épiderme pluristratifié.

12. Utilisation selon l'une quelconque des revendications 1 à 9, **caractérisée en ce que** le mimétique du LIF est choisi parmi tout agoniste du récepteur du LIF (LIF-R), tout actif ou extrait cellulaire capable d'activer la gp130 et/ou les voies de signalisation des Jak/Stat et Ras/Map kinases, en particulier l'expression et/ou l'activité de Stat3.

13. Utilisation selon l'une des revendications 1 à 12, **caractérisée en ce que** la composition est destinée à une administration orale ou une application topique sur la peau et/ou les muqueuses ou sur le cuir chevelu.

14. Utilisation selon l'une des revendications 1 à 13, **caractérisée en ce que** la quantité efficace de LIF, d'un analogue du LIF, d'un mimétique du LIF, d'un produit capable de stimuler l'expression du LIF endogène ou leur mélange, présente dans la composition, est comprise entre 10⁻¹² % et 1 % du poids total de la composition, préférentiellement entre 10⁻⁹ % et 0.1%, encore plus préférentiellement entre 10⁻⁷ % et 0.01% du poids total de la composition.

15. Composition adaptée à une application topique sur la peau comprenant, dans un milieu physiologiquement acceptable, au moins une quantité efficace de LIF, d'un analogue du LIF, d'un mimétique du LIF tels que définis dans l'une quelconque des revendications 10 à 12, ou leurs mélanges.

16. Composition selon la revendication 15, **caractérisée en ce que** la quantité efficace de LIF, d'un analogue du LIF, d'un mimétique du LIF, d'un produit capable de stimuler l'expression du LIF endogène ou leurs mélanges, est comprise entre 10⁻¹² % et 1 % du poids total de la composition, préférentiellement entre 10⁻⁹ % et 0.1%, encore plus préférentiellement entre 10⁻⁷ % et 0.01% du poids total de la composition.

17. Composition selon l'une quelconque des revendications 15 ou 16, **caractérisée en ce qu'**il s'agit d'une composition cosmétique ou dermatologique comprenant en outre au moins un actif hydrophile ou lipophile choisi parmi les agents hydratants, les agents apaisants, les agents dépigmentants, les agents anti-glycation, les inhibiteurs de NO-synthase, les agents stimulant la synthèse de macromolécules dermiques ou épidermiques et/ou empêchant leur dégradation, les agents stimulant la prolifération des fibroblastes et/ou des kératinocytes ou stimulant la différenciation des kératinocytes, les agents dermo-décontractants, les agents tenseurs, les agents anti-pollution et/ou anti-radicalaires, les agents photoprotecteurs, les agents réparateurs et/ou cicatrisants, les agents antipelliculaires et leurs mélanges.

18. Procédé cosmétique de traitement des peaux sèches et/ou gercées et/ou âgées, **caractérisé en ce que** l'on applique sur la peau ou les muqueuses une composition cosmétique telle que définie dans l'une des revendications 15 à 17.

19. Procédé cosmétique de traitement de la chute des cheveux, **caractérisé en ce que** l'on applique sur le cuir chevelu une composition cosmétique telle que définie dans l'une des revendications 15 à 17.
